# EUROPEAN PATENT APPLICATION

(11) **EP 4 382 145 A1**
(43) Date of publication of application: **12.06.2024**
(21) Application number: 22212197.2
(22) Date of filing: 08.12.2022
(51) Int. Cl.: A61M 1/06

(54) **A NIPPLE STIMULATION UNIT**

(71) Applicant: Koninklijke Philips N.V., 5656 AG Eindhoven (NL)
(72) Inventor: LIPSCH, Job, Eindhoven (NL); PALERO, Jonathan Alambra, Eindhoven (NL); BOURQUIN, Yannyk Parulian Julian, Eindhoven (NL); WIJNOLTZ, Anna Louise, Eindhoven (NL); VAN DER KOOI, Johannes Tseard, Eindhoven (NL); DE VREEDE, Jasper, Eindhoven (NL); SEGAAR, ucja El bieta, Eindhoven (NL); VAN DEN BIJGAART, Adrianus Wilhelmus Dionisius Maria, Eindhoven (NL); VAN BENTUM, Jesper William, Eindhoven (NL)
(74) Representative: Philips Intellectual Property & Standards

(57) **Abstract**

A nipple stimulation unit is used for promoting milk expression. It comprises a nipple contacting arrangement for contacting a nipple having one or more flexible arms each having a distal end which is configured, in use, to be directed towards the nipple. A respective vibration element is mounted along each of the flexible arms for delivering vibration to the distal end of the flexible arm.

## Description

### FIELD OF THE INVENTION

This invention relates to nipple stimulation, for example to be used as part of a breast pump or other milk collection device.

### BACKGROUND OF THE INVENTION

Breast pumps are used by breast feeding women to extract milk from their breast such that the extracted milk can be fed to their babies at a later time.

It is well known that the best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

To use a breast pump, the breast is typically placed into a funnel-shaped cup and a vacuum is applied such that milk is extracted. A motorized breast pump typically has two operating modes, namely a stimulation mode and an extraction mode. The operation of a typical breast pump makes use of a cyclic pressure waveform, typically a negative pressure or vacuum, which is applied to the breast and nipple within a breast shield (or a pair of breast shields) to draw the milk from the nipple into a collection container. The typical pressure profiles oscillate between a maximum negative pressure and then return to atmospheric pressure at the end of each cycle.

During the stimulation mode, the milk ejection reflex is stimulated. This for example makes use of a relatively high frequency cyclic pressure waveform such as 2 Hz. Once milk comes out of the breast it is advised to switch to the extraction mode, which has a lower frequency (typically around 1 Hz) and higher intensity pumping (i.e. lower under pressure) for more effective milk extraction.

When the breast pump is activated, a pressure source such as a vacuum pump inside the breast pump generates a vacuum and the stimulation mode can be started. The vacuum pump is typically driven by an electric motor. For portable breast pumps, these motors are driven by battery power. There are also breast pumps using manually driven mechanical pumps.

Recently, there is a trend towards wearable breast pumps. These devices are generally in a smaller form factor and can be fully worn on the user's body, allowing the user to move around freely. Some of these are small enough to fit into a user's bra. A motor, battery and milk container are for example integrated to form a single unit.

Additionally, passive milk collectors exist, which can be placed on the breast and often allow the user to apply a static under pressure to hold the device in place and help in extracting milk. These collectors are for example meant to be used when breastfeeding to collect milk from the nonbreastfeeding breast. The stimulation provided by the infant generates a milk ejection reflex on both breasts, and a simple passive vacuum is often enough to extract (some) milk.

Unfortunately, these milk collectors cannot be used at other moments when the baby is not around since stimulation from the baby is needed to create a milk ejection reflex (MER) to start the milk flow.

Nipple stimulation based on mechanical means including vibration to stimulate MER is known in the art. For example it is known to apply vibration to promote the MER, for example as disclosed in EP 4 029 540. Creation of MER by stimulation without a baby results in extra milk being collected with the milk collectors at any suitable time.

However, stimulation requires good contact to the nipples for effective stimulation. Nipple shapes and sizes vary between persons as well as dynamically changing during milk expression. These variances in nipple dimensions result in inconsistent and unreliable nipple stimulation.

There is thus a need for nipple stimulation that is able to accommodate different sizes and shapes of nipple, ensuring good contact during milk expression.

### SUMMARY OF THE INVENTION

The invention is defined by the claims.

According to examples in accordance with an aspect of the invention, there is provided a nipple stimulation unit for promoting milk expression, comprising:
a nipple contacting arrangement for contacting a nipple, the nipple contacting arrangement comprising a set of one or more flexible arms each having a distal end which is configured, in use, to be directed towards the nipple such that the nipple contacting arrangement can make contact with a range of nipple sizes; and
a vibration system comprising a set of one or more vibration elements, with a respective vibration element mounted along each of the flexible arms for delivering vibration to the distal end of the flexible arm.

The nipple contacting arrangement transfers vibrations to the nipple by means of a vibration element mounted along at least one flexible arm. The design of the flexible arm or arms creates a bias towards the nipple once it has been flexed. In particular, deformation of the flexible arm or arms away from a default rest state creates a restoring force. The nipple contacting arrangement is for example for contacting the sides and the base of the nipple. This provides effective stimulation without blocking the milk duct exits.

This system provides a cost effective, small and quiet way of generating the milk ejection reflex by applying a mechanical oscillation or vibration to the nipple/areola region.

The vibration element of each flexible arm is preferably located at the distal end of the flexible arm. Thus, the vibration elements are at the location where the flexible arm or arms make contact with the nipple.

The nipple stimulation unit for example comprises a set of at least three flexible arms, wherein the distal ends of the flexible arms lie on the path of a closed shape which is configured, in use, to be positioned around the nipple, wherein the flexible arms bias the path inwardly (if they have previously been deformed outwardly from a rest state).

The closed path thus surrounds the nipple and is able to adopt a size which is suitable for making contact with a range of possible nipple sizes. The path is for example configurable to form a ring with a range of diameters which includes the range 12 mm to 18 mm. The path for example is a circle with a diameter of 4 mm when no nipple is present, and opens to accommodate the inserted nipple to a size which matches the size (diameter) of the nipple. The restoring force (urging the ring back to its 4 mm diameter) is then applied to the nipple.

The distal ends of the flexible arms for example comprise spherical, ellipsoidal, kidney bean-shaped, or U-shaped bodies. The distal ends are for example are spherical with a diameter 8 mm.

The nipple stimulation unit may comprise a frame for coupling together proximal ends of the set of flexible arms. This frame serves as a mounting feature. It for example mounts the nipple contacting arrangement and the vibration system to a milk collection chamber.

The vibration elements for example each comprise:
an electrical actuator; or
a piezoelectric actuator; or
a mechanical actuator

An electrical actuator may comprise a motor with an eccentric rotating mass, a motor actuating planetary gears or a motor with a cam or similar arrangement for applying a varying contact depth or an intermittent contact and release. A linear resonance actuator may for example be used.

The motor in the example above may be replaced with a manually actuated rotating unit. A mechanical actuator in the form of a spring powered mechanism may be used.

A vibration frequency of the vibration system may be in the range 1 to 250 Hz.

When there are multiple arms, they may vibrate in synchronism, hence follow a same pattern at the same time, or they may vibrate in a sequence, hence follow a pattern one after the other, or they may be driven with independent patterns in terms of frequency and amplitude.

A vibration amplitude of the vibration system is for example less than 2 mm. This is found to be sufficient to provide the required stimulation to promote the milk ejection reflex.

A stiffness of the set of arms may be tunable. The stiffness then determines the restoring force that is applied. This may be achieved with electroactive polymers, or by means of a pressure chamber, or based on temperature change. The pressure exerted on the nipple may thereby be controllable.

The nipple stimulation unit may further comprise a contact pressure sensor. This monitors the pressure applied and enables a feedback loop to be provided for controlling the tunable stiffness.

The invention also provides a milk expression system comprising:
the nipple stimulating unit as defined above; and
a milk collection chamber for positioning over the nipple for collecting expressed milk.

Thus, the expression system stimulates milk expression and provides the collection of milk. The nipple stimulating unit is preferably internal to the milk collection chamber.

The milk expression system may further comprise a pressure source for generating a pressure below atmospheric pressure in the milk collection chamber. This (static) negative pressure may be delivered to the chamber directly or indirectly by means of a membrane. The reduced pressure further stimulates milk expression.

The invention also provides a breast pump incorporating the milk expression system, and also a milk collection system incorporating the milk expression system.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a known breast pump system;
Fig. 2 shows a wearable breast pump;
Fig. 3 shows a nipple stimulation unit in accordance with the invention, applied to a milk collector; and
Fig. 4 shows one example of the nipple stimulation unit in perspective view.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a nipple stimulation unit for promoting milk expression. It comprises a nipple contacting arrangement for contacting a nipple, having one or more flexible arms each having a distal end which is configured, in use, to be directed towards the nipple. A respective vibration element is mounted along each of the flexible arms for delivering vibration to the distal end of the flexible arm.

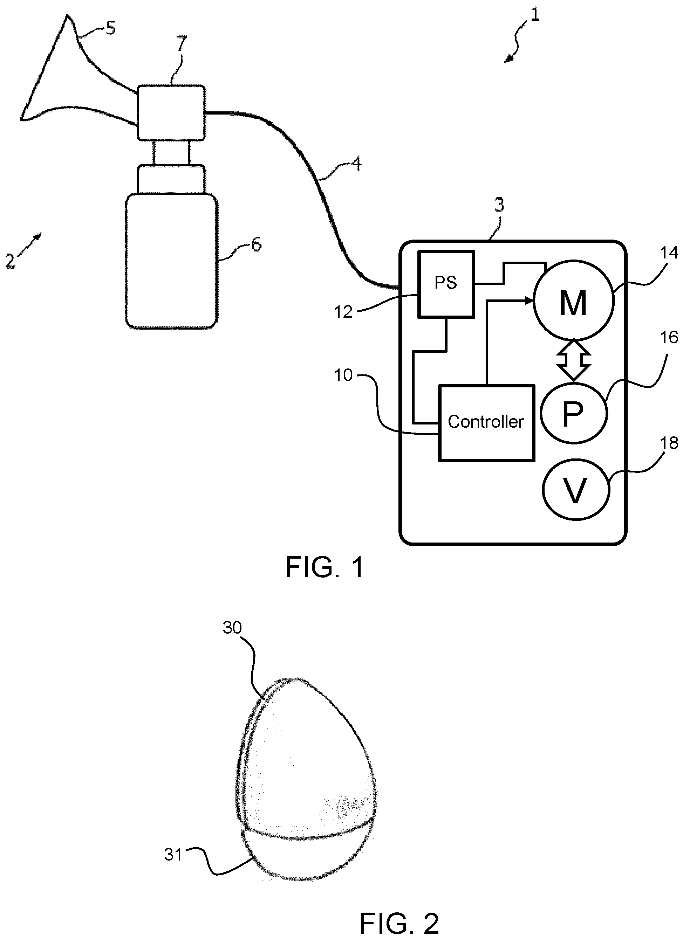

Fig. 1 shows a known breast pump system 1, comprising an expression unit 2 and a drive arrangement for controlling the expression function of the breast pump.

In the example shown, the drive arrangement comprises an electric pump arrangement 3 which is connected via a tube 4 to the expression unit 2. The pump arrangement includes various components in addition to a pump impeller and the pump motor, so may be considered to be a general operating unit.

The expression unit 2 is formed with a main body 7, a funnel 5 (known as a breast shield) for receiving a breast of a user and a milk collection container 6 for collecting the expressed milk. The funnel 5 and the container 6 are connected to the main body 7. The main body 7 comprises a vacuum chamber. A flexible membrane known as the diaphragm is located in the vacuum chamber. The diaphragm prevents expressed milk from flowing into the tube 4 leading to the pump arrangement unit 3.

The operating unit, including the pump arrangement 3, may instead be directly mounted and connected to the main body 7. In this case, the diaphragm prevents expressed milk from flowing directly into the pump arrangement 3.

The pump arrangement 3 comprises a controller 10, a power source 12, a motor 14 and a vacuum pump 16 (i.e. the impeller driven by the motor 14). A membrane pump (displacement pump) may instead be used. The controller controls 10 the operation of the power source 12, motor 14 and vacuum pump 16. The pump arrangement 3 further comprises a solenoid valve 18.

In use, the vacuum pump 16 applies a vacuum to the diaphragm located in the main body 7 so that it deforms. The diaphragm deforms to create a vacuum in the funnel 5, when it is sealed to the breast, which in turn applies a vacuum to the breast which enables milk to be expressed.

The vacuum is applied to the breast at intervals. That is, a pressure differential is applied on a cyclic basis. After a vacuum has been established, the pressure from the vacuum is released by the use of the solenoid valve which is temporarily opened. The solenoid valve is an electromechanically operated valve configured to open and close an air passage that connects the vacuum side of the vacuum pump to ambient air such that when the solenoid valve is closed, the vacuum pump generates a vacuum in the expression unit which enables milk to be expressed from the breast of a user. When the solenoid valve is opened, the vacuum generated by the vacuum pump is released as ambient air flows towards the vacuum or negative pressure created by the vacuum pump such that the pressure exerted on the breast of a user is partially or completely reduced.

This is a basic description of the known operation of a standard breast pump system.

There are also wearable breast pumps, which are for example for mounting within a feeding bra. All of the drivetrain components described above are then formed within an outer enclosure which fits over the breast. A top part contains the drivetrain (pump, motor, controller) and a bottom part forms the collection container. This enables breast pumping to be performed more discretely and leaves the hands free to do other things.

There are also other types of wearable breast pump whereby the expression unit is fixed to a breast and the pump unit and/or milk collection container are situated elsewhere on the body of a user.

Fig. 2 shows a wearable breast pump, comprising an expression kit 30 attached to a respective milk collection container 31.

The invention relates to stimulation of the nipple to promote the milk ejection reflex. This stimulation may be used to avoid the need for a particular cyclic pressure waveform. For example, there may be a static negative pressure applied. This then avoids the need for components such as vacuum pumps, motors, valves, and large batteries. Thus, the nipple stimulation unit may be used as part of a device (breast pump or milk collector) which avoids the problems or large cost, size, weight, and noiselevel. It may be part of a milk collection system or a simple breast pump without needing a cyclic pressure waveform. However, the nipple stimulation approach may be used in a normal breast pump (with a cyclic pressure waveform) as well.

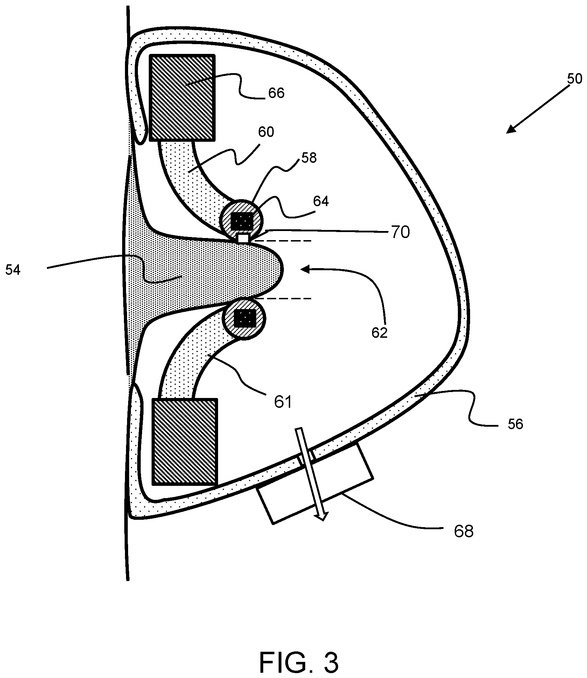

Fig. 3 shows a nipple stimulation unit used within a milk collection device 50.

The milk collection device 50 is designed to provide robust and effective mechanical stimulation of different nipple sizes and shapes. The device 50 functions as a vibration stimulation unit, for providing contact to the nipple 54 and applying mechanical vibration for stimulating MER.

The nipple stimulation unit has a nipple contacting arrangement for contacting a nipple. The nipple contacting arrangement comprises a set of one or more flexible arms 60, 61 each having a distal end 58 which is configured, in use, to be directed towards the nipple 54 such that the nipple contacting arrangement can make contact with a range of nipple sizes.

The arm or arms 60, 61 have a default rest configuration, and the presence of the nipple causes the arm or arms to flex away from this rest position. The inherent elasticity of the arm or arms 60, 61 results in a restoring force being exerted against the nipple. Thus, the arms are flexible but elastically deformable.

The distal ends of the flexible arms have a smooth surface for contacting the nipple. For example they may comprise spherical, ellipsoidal, kidney bean-shaped, or U-shaped bodies.

A vibration system comprises a set of vibration elements, with a respective vibration element 64 mounted along each of the flexible arms for delivering vibration to the distal end of the flexible arm. The vibration element can generate reciprocating movement in one or several directions with respect to the nipple. Each vibration element may comprise a single actuator or it may comprise multiple actuators oriented differently and controlled in unison or independently.

The vibration may have a vibration axis in a transverse or longitudinal direction (relative to the nipple axis) or a combination of both. The vibration may applied in a continuous mode or in a pulsed mode (wherein each pulse comprises a set of vibrations). A pulsed mode may be perceived as more comfortable when high frequency vibrations are used.

The distal end 58 preferably makes contact with the sides and base of the nipple and the areola. The distal end is made of material that conducts the mechanical vibration from the vibration element 64 to the nipple. The vibration element 64 is housed on, or embedded in, the flexible arm.

In the example shown, there is an upper flexible arm 60 and a lower flexible arm 61. As a minimum, there may be a single flexible arm. The space between that arm and a fixed support may then be adjusted by the flexibility and deformation of the flexible arm. With two flexible arms as shown, the space between them is adjusted by the flexibility and deformation of the flexible arms, such that the nipple is pinched between them,

There may be a set of three or more arms. In this way, the distal ends of the flexible arms may form a path which is configured, in use, to be positioned around the nipple. The path is for example a circle, but it could be any closed shape. Indeed a set of three points may be considered to lie on a circle or define a triangle. In preferred examples, the distal ends are evenly angularly spaced around a circle. The bias of the flexible arms (back to their rest state) urges the ring path inwardly to contact the nipple. Such a ring path is for example configurable with a range of diameters which includes the range 12 mm to 18 mm.

The arm structure of at least three arms then surrounds an effective nipple opening 62, wherein the nipple opening 62 is configured such that the distal ends 58 contact the sides or base of the nipple, or the areola and not the tip where the opening of the milk ducts might be blocked. The flexibility of the flexible arms ensures that the nipple opening 62 is able to vary in size to accommodate a wide range of nipple diameters.

The vibration units 64 generate vibration by electrical means, e.g., piezo-actuators, vibration motors, etc., or by mechanical means, e.g., a windup spring motor or by movement-induced vibration of a spring-loaded ring;

A frame 66 holds together the proximal ends of the flexible arms 60, 61 and functions as a support for the arm arrangement.

A milk collection unit 56 covers the nipple stimulation unit, for collecting milk.

The distal ends are formed of a material that is soft enough for nipple contact and the arm or arms 60, 61 are flexible enough to deflect towards the nipple, but when contact is made the pressure is not so high as to cause irritation and block the milk ducts.

Each vibration element 64, when in use and in contact to the nipple, for examples provides vibration to the nipples with a frequency in the range between 1 Hz to 250 Hz and with amplitude of less than 2 mm. The stimulation of the nipple for promoting milk expression is influenced by several aspects such as vibration frequency, vibration amplitude, and the location of stimulation (e.g., side of nipples, areola, top of the nipple as long as that does not interfere with milk coming out of the ducts).

In one example, miniature mechanical vibrators may be embedded in the distal ends of three arms, and spherical nipple contacting elements are formed at the distal ends. The arms for example have a length in the range 10 mm to 20 mm (e.g. 15 mm) and the spherical nipple contacting elements have a diameter in the range 5 mm to 10 mm (e.g. 8 mm). The minimum nipple opening diameter is for example 4 mm and the flexible arms allow opening to up to 18 mm after insertion of a nipple length. The configuration allows insertion of a nipple of the typical range of nipple lengths, e.g. 7 mm to 14 mm.

The volume of the milk collecting chamber may be provided with a negative pressure (i.e. below atmospheric pressure). This may be a static negative pressure, which is therefore more simple to generate than a cyclic pressure waveform.

By static pressure is meant that only a single pressure level is applied and there is no active pressure modulation over time. The pressure may however change over time due to pressure leakage or the filling of the milk container, but there is in this example no active modulation of an applied pressure.

A negative pressure source 68 for this purpose may comprise a pump (electrical or mechanical) or it may be a compressible chamber for manually creating an under pressure, or it may even be implemented by the milk collection container 50 itself.

The distal end of each flexible arm may be integral with the arm or it may be a separate element.

In an example, the stiffness of arrangement of flexible arms can be tuned, e.g., using electroactive polymers, a pressure chamber, or based on temperature change. The stiffness may adapted in other ways, such as by blowing in air or fluid to a hollow cavity of the flexible arms thereby to change their elasticity.

Another option is to provide an actuator for active rather than entirely passive movement of the arms, or by manually changing a position of the flexible arms (and hence a distance to the nipple) by the user, e.g., using a screw-like mechanism. An alternative mechanical adjustment is for example to adjust the size of the frame 66, for example by having the frame deformable or inflatable, to adjust the nipple opening.

The pressure exerted by the arrangement of flexible arms on the nipple may in these various ways be controllable.

A contact pressure sensor 70 may also be integrated into the distal ends of one or more arms, or a pressure or strain sensor may be integrated into the arms or provided at the frame, to monitor the pressure applied and provide a feedback loop.

The stiffness of the arms is for example around 0.005 N/mm (e.g. in the range 0.015 - 0.02 N/mm). The selection of the arm stiffness and size and configuration determines the mechanical performance of the arm arrangement.

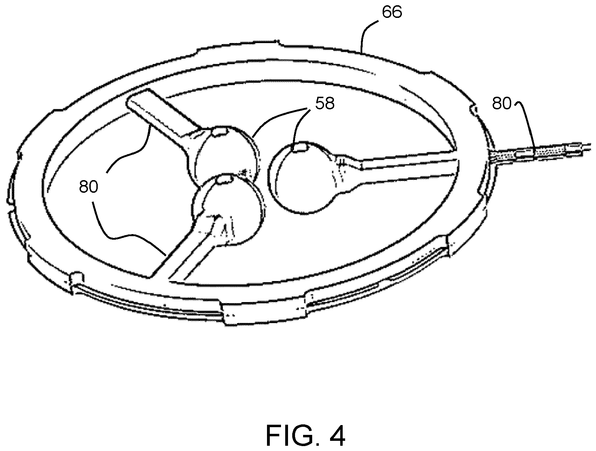

Fig. 4 shows one example of the nipple stimulation unit in perspective view.

It comprises an arrangement of three arms 80 with spherical distal ends 58. The arms 80 are overmolded over the vibration elements and the electrical wired connections 82 to the vibration elements. These wired connections extend around the frame 66 and along the arms to reach the vibration elements. The vibration elements are thus embedded in the spherical distal ends.

Fig. 4 shows the relaxed state for example with a 4 mm opening defined between the distal ends 58. When the device is applied to the breast so that a nipple is present, the arms deflect to increase the size of the opening as shown in Fig. 3.

Each arm for example comprises a pair of parallel arm portions with a slot between them for the wire connection. The slot lies is the neutral plane so the wiring does not contribute significantly to the stiffness of the arms.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A nipple stimulation unit for promoting milk expression, comprising:
a nipple contacting arrangement (58,60) for contacting a nipple, the nipple contacting arrangement comprising a set of one or more flexible arms (60) each having a distal end (58) which is configured, in use, to be directed towards the nipple such that the nipple contacting arrangement can make contact with a range of nipple sizes; and
a vibration system comprising a set of one or more vibration elements (64), with a respective vibration element (64) mounted along each of the flexible arms (60) for delivering vibration to the distal end (58) of the flexible arm.

2. The nipple stimulation unit of claim 1, wherein the vibration element (64) of each flexible arm is located at the distal end of the flexible arm.

3. The nipple stimulation unit of claim 1 or 2, comprising a set of at least three flexible arms (60, 61), wherein the distal ends of the flexible arms lie on the path of a closed shape which is configured, in use, to be positioned around the nipple, wherein the flexible arms bias the path inwardly.

4. The nipple stimulation unit of claim 3, wherein the path is configurable to form a ring with a range of diameters which includes the range 12 mm to 18 mm.

5. The nipple stimulation unit of any one of claims 1 to 4, wherein the distal ends (58) of the flexible arms comprise spherical, ellipsoidal, kidney bean-shaped, or U-shaped bodies.

6. The nipple stimulation unit of any one of claims 1 to 5, comprising a frame (66) for coupling together proximal ends of the set of flexible arms.

7. The nipple stimulation unit of any one of claims 1 to 6 wherein the vibration elements (64) each comprise:
an electrical actuator; or
a piezoelectric actuator; or
a mechanical actuator

8. The nipple stimulation unit of any one of claims 1 to 7, wherein a vibration frequency of the vibration system is in the range 1 to 250 Hz.

9. The nipple stimulation unit of any one of claims 1 to 8, wherein a vibration amplitude of the vibration system is less than 2 mm.

10. The nipple stimulation unit of any one of claims 1 to 9, wherein a stiffness of the set of arms is tunable.

11. The nipple stimulation unit of claim 10, further comprising a contact pressure sensor (70).

12. A milk expression system comprising:
the nipple stimulating unit of any one of claims 1 to 11; and
a milk collection chamber for positioning over the nipple for collecting expressed milk.

13. The milk expression system of claim 12, further comprising a pressure source (68) for generating a pressure below atmospheric pressure in the milk collection chamber.

14. A breast pump incorporating the milk expression system of claim 12 or 13.

15. A milk collection system incorporating the milk expression system of claim 12 or 13.
